# EUROPEAN PATENT APPLICATION

(11) **EP 3 115 461 A1**
(43) Date of publication of application: **11.01.2017**
(21) Application number: 15175659.0
(22) Date of filing: 07.07.2015
(51) Int. Cl.: C12P 17/00, C12P 19/28

(54) **METHOD FOR THE SYNTHESIS OF PENTOSTATIN**

(71) Applicant: Synbias Pharma AG, 8200 Schaffhausen (CH)
(72) Inventor: Zabudkin, Oleksandr, 76593 Gerusbach (DE); Schickaneder, Christian, 91207 Lauf an der Pegnitz (DE); Matviienko, Iaroslav, 68305 Mannheim (DE); Sypchenko, Volodymyr, 68168 Mannheim (DE)
(74) Representative: Kopf Westenberger Wachenhausen Patentanwälte PartG mbB

(57) **Abstract**

The present invention relates to a method for the stereo-selective production of pentostatin comprising an enzymatic transglycosylation reaction between an imidazo-[4,5-*d*]-[1,3]diazepine compound and a 2'-deoxyribonucleotide, followed by an ruthenium-catalyzed asymmetric transfer hydrogenation.

## Description

### FIELD OF THE INVENTION

The present invention relates to a method for the stereo-selective production of pentostatin comprising an enzymatic transglycosylation reaction between an imidazo-[4,5-*d*]-[1,3] diazepine compound and a 2'-deoxyribonucleotide, followed by an ruthenium-catalyzed asymmetric transfer hydrogenation. The method results in the production of pentostatin in high yield and with a > 99% excess of the desired β-anomer.

### BACKGROUND OF THE INVENTION

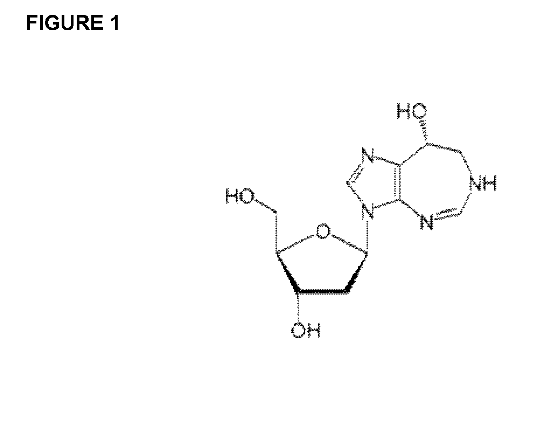

Pentostatin is a cytostatic purine analogue acting as one of the most potent inhibitors of adenosine deaminase that is used for the treatment of various types of lymphoproliferative disorders. The systematic name of pentostatin is (R)-3-((2R,4S,5R)-4-hydroxy-5-(hydroxylmethyl)tetrahydrofuran-2-yl)-3,6,7,8-tetrahydro-imidazo[4,5-d][1,3]diazepin-8-ol) and its chemical structure is illustrated in **FIG.1****.**

Currently, the primary method for the large-scale production of pentostatin relates to the fermentation of *Streptomyces anlibilicus* NRRL 3238 cultures. However this method has several serious drawbacks including a very low yield of the final product (~13 g from 1000 L of fermentative broth) as well as a complicated and expensive chromatography purification process (Woo, P.W.K. et al. (1974) J. Heterocycl. Chem. 11, 641-645).

The total chemical synthesis of pentostatin poses a challenge since the molecule contains (i) a unique and unstable heterocyclic base, (ii) a 2-deoxy sugar that defies attempts at stereo-controlled glycosylation to favor the β-anomer, and (iii) a central chiral hydroxyl group. The merit of any chemical transformation is measured by its resolutions to these three key difficulties.

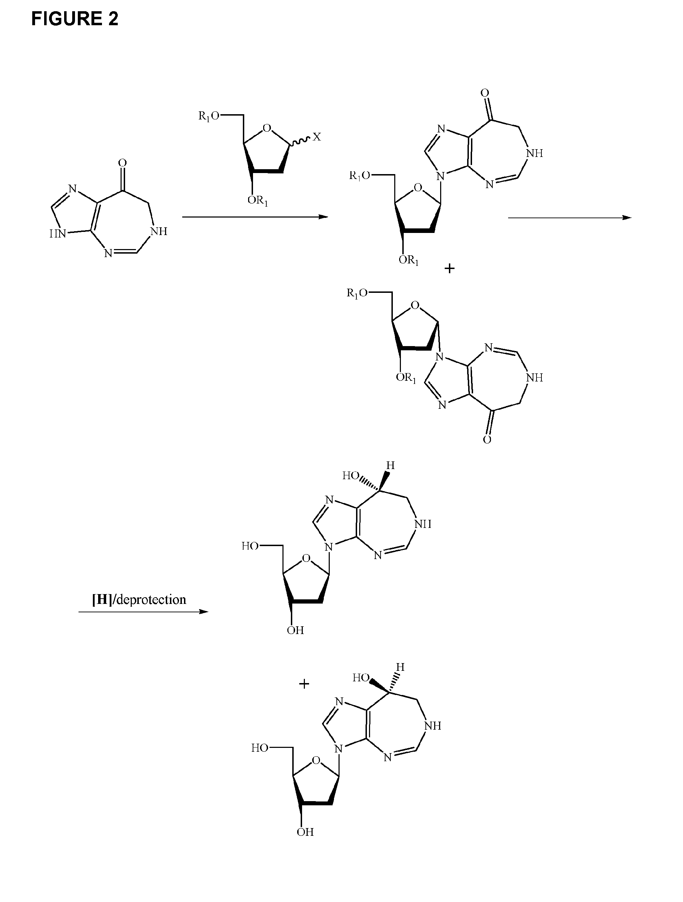

The first synthesis scheme of pentostatin was established in 1979 (Baker, D.C., and Putt, S.R. (1979) J. Am. Chem. Soc. 101, 6127-6128). The synthesis route is schematically depicted in **FIG. 2** and included a total of eleven steps. It involves the production of an imidazo[4,5-*d*][1,3]diazepine heterocyclic compound followed by non-selective glycosidation (with the portion of the desired β-anomer representing only 14%) and reduction. The overall yield is only 1.6% (as compared to the staring materials),

Subsequently, several modifications and improvements of this method were established (see, e.g., Chen, B.C. et al. (2002) Tetrahedron Lett. 43, 1595-1596; Ho, J.Z. et al. (2003) J. Org. Chem. 68, 109-114), all of which are still hampered by the rather low yield due to the lack of stereo-specificity during individual synthesis steps.

WO 2005/027838 A2 discloses a synthesis scheme for the production of pentostatine comprising a ring expansion of an O-C-N functionality in a hypoxanthine or 2-deoxyinosine derivative, resulting in an improved yield. However, due to its overall chemical complexity this synthesis route is also not suitable for the large-scale production of pentostatin.

Hence, there is still an ongoing need for improved methods for the synthesis of pentostatin that overcome the limitations of the established synthesis routes. In particular, there is a need for a less laborious and cost-efficient method for the large-scale production of pentostatin, thus improving stereo-specificity of the individual reaction steps in favor of the desired β-anomer.

Accordingly, it is an object of the present invention to provide an improved method for the synthesis of pentostatin.

### SUMMARY OF THE INVENTION

In one aspect, the present invention relates to a method for the stereo-selective synthesis of the pentostatin, comprising:
(i) reacting an imidazo-[4,5-*d*]-[1,3]diazepine compound having formula (I) and a 2-deoxy-ribonucleoside, 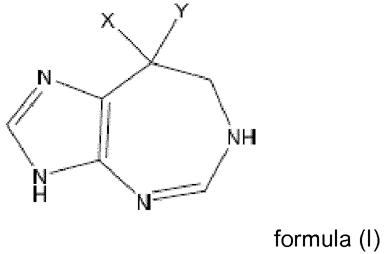 with X and Y being independently selected from H and OH or together representing a keto group =O; and
(ii) reducing the 8-keto pentostatin derivative obtained in step (i) by ruthenium-catalyzed asymmetric transfer hydrogenation;
   wherein the reaction in step (i) is enzymatically catalyzed by nucleoside deoxyribosyltransferase.

In a preferred embodiment, the 2-deoxy-ribonucleoside used is 2-deoxy-uridine. In another preferred embodiment, the imidazo-[4,5-*d*]-[1,3]diazepine compound used is 6,7-dihydroimidazo-[4,5-*d*]-[1,3]diazepin-8(3*H*)-one.

In a particular embodiment, the 2-deoxy-ribonucleoside is used in an amount of 1.5 to 10 molar equivalents of the amount of the imidazo[4,5-*d*] [1,3]diazepine compound. Preferably, the 2-deoxy-ribonucleoside is used in an amount of 4 to 7 molar equivalents of the amount of the imidazo[4,5-*d*][1,3]diazepine compound.

In another particular embodiment, the reaction in step (i) is performed at a temperature between 10°C and 50°C. Preferably, the reaction in step (i) is performed at a temperature between 25°C and 35°C.

In another particular embodiment, the reaction in step (i) is performed at a pH value between 6.0 and 9.0. Preferably, the reaction in step (i) is performed at a pH value between 7.5 and 8.0.

In a preferred embodiment, the reaction in step (ii) is catalyzed by RuCI(p-cymene)[(R,R)-Ts-DPEN]. Particularly preferably, the reaction in step (ii) is performed in a mixture of triethylamine and formic acid.

### BRIEF DESCRIPTION OF THE DRAWINGS

**FIGURE 1** illustrates the chemical structure of (R)-3-((2R,4S,5R)-4-hydroxy-5-(hydroxymethyl)tetrahydrofuran-2-yl)-3,6,7,8-tetrahydroimidazo[4,5-d][1,3]diazepin-8-ol) (i.e., pentostatin).
**FIGURE 2** illustrates a representative synthesis scheme for the production of pentostatin being established in the art.

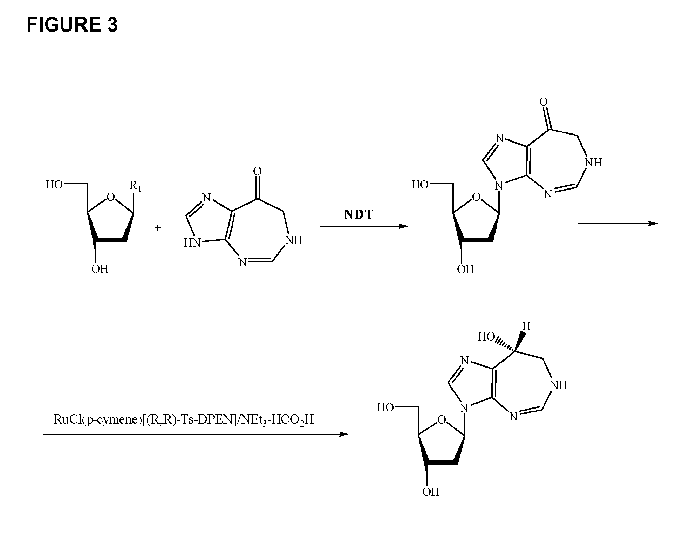

**FIGURE 3** illustrates a representative synthesis scheme for the stereo-selective production of pentostatin according to the present invention.

### DETAILED DESCRIPTION OF THE INVENTION

The present invention is based on the unexpected finding that combining an enzymatic transglycosylation reaction of an imidazo-[4,5-*d*]-[1,3]diazepine compound with a 2-deoxy-ribonucleoside and a subsequent ruthenium-catalyzed asymmetric transfer hydrogenation, performed on the unprotected 8-keto pentostatin derivative obtained, results in a significant increase in yield of final product (about 4-fold as compared to established methods) by shifting stereo-selectivity of the overall reaction towards a huge excess of, and preferably completely to the desired β-anomer.

The present invention will be described in the following with respect to particular embodiments and with reference to certain drawings but the invention is to be understood as not limited thereto but only by the appended claims. The drawings described are only schematic and representative and are to be considered non-limiting.

Where the term "comprising" is used in the present description and claims, it does not exclude other elements or steps. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Where an indefinite or definite article is used when referring to a singular noun e.g. "a", "an" or "the", this includes a plural of that noun unless specifically stated otherwise.

In case, numerical values are indicated in the context of the present invention the skilled person will understand that the technical effect of the feature in question is ensured within an interval of accuracy, which typically encompasses a deviation of the numerical value given of ± 10%, and preferably of ± 5%.

Furthermore, the terms first, second, third, (a), (b), (c), and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used. The following terms or definitions are provided solely to aid in the understanding of the invention. These definitions should not be construed to have a scope less than understood by a person of ordinary skill in the art.

In one aspect, the present invention relates to a method for the stereo-selective synthesis of the pentostatin, comprising:
(i) reacting an imidazo-[4,5-*d*]-[1,3]diazepine compound having formula (I) and a 2-deoxy-ribonucleoside, 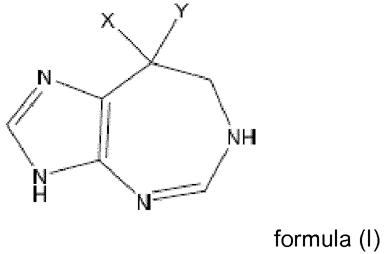 with X and Y being independently selected from H and OH or together representing a keto group =O; and
(ii) reducing the 8-keto pentostatin derivative obtained in step (i) by ruthenium-catalyzed asymmetric transfer hydrogenation;
   wherein the reaction in step (i) is enzymatically catalyzed by nucleoside deoxyribosyltransferase.

The transglycosylation reaction in step (i) is catalyzed by nucleoside deoxyribosyltransferase (i.e., nucleoside:purine(pyrimidine) deoxy-D-ribosyltransferase having the EC number 2.4.2.6). This enzyme catalyzes the general reaction: 2-deoxy-D-ribosyl-base¹ + base² = 2-deoxy-D-ribosyl-base² + base¹. Typically, the enzyme employed is a recombinant enzyme, being commercially available from various suppliers.

In a preferred embodiment, the imidazo-[4,5-*d*]-[1,3]diazepine compound used is 6,7-dihydroimidazo-[4,5-*d*]-[1,3]diazepin-8(3*H*)-one.

The imidazo-[4,5-*d*]-[1,3]diazepine compound having formula (I) may be reacted with any 2-deoxy-ribonucleosides, such as naturally occurring 2-deoxy-ribonucleosides as well as artificially produced analogs thereof. Examples of suitable 2-deoxy-ribonucleosides include 2-deoxy-adenosine, 2-deoxy-cytosine, 2-deoxy-guanine, 2-deoxy-uridine, 2-deoxy-thymidine, 2-deoxy-inosine, and 5-aza-derivatives thereof.

In a preferred embodiment, the 2-deoxy-ribonucleoside used is 2-deoxy-uridine.

Since the transglycosylation reaction in step (i) is reversible the amount of 2-deoxy-ribonucleoside used is typically higher than the amount of the imidazo[4,5-*d*] [1,3]diazepine compound, that is, the 2-deoxy-ribonucleoside is used in a molar excess.

In a particular embodiment, the 2-deoxy-ribonucleoside is used in an amount of 1.5 to 10 molar equivalents of the amount of the imidazo[4,5-*d*] [1,3]diazepine compound. Preferably, the 2-deoxy-ribonucleoside is used in an amount of 4 to 7 molar equivalents of the amount of the imidazo[4,5-*d*][1,3]diazepine compound. For example, the 2-deoxy-ribonucleoside is used in an amount of 4.0, 4.2, 4.4., 4.6, 4.8, 5.0, 5.2, 5.4, 5.6, 5.8, 6.0, 6.2, 6.4, 6.6, 6.8 or 7.0 molar equivalents of the amount of the imidazo[4,5-*d*][1,3]diazepine compound, with a molar excess of 5.6, 5.8 or 6.0 being particularly preferred.

Typically the reaction in step (i) is performed at a temperature between 10°C and 50°C. Preferably, it is performed at a temperature between 25°C and 35°C. For example, the reaction in step (i) may be performed at a temperature of 25°C, 28°C, 30°C, 32°C or 35°C.

Furthermore, the reaction in step (i) typically is performed at a pH value between 6.0 and 9.0. Preferably, it is performed at a pH value between 7.5 and 8.0. For example, the reaction in step (i) may be performed at a pH of 7.5, 7.6, 7.7, 7.8, 7.9 or 8.0.

Due to the stereospecificity of enzymatic transglycosylation, the only product is the desired β-anomer, that is, the 8-keto pentostatin derivative, which further results in a significant simplification of the synthesis scheme as there is no need for additional purification steps in order to remove the α-anomer. The yield of the 8-keto pentostatin derivative obtained is thus up to two-fold higher as compared to established glycosylation methods.

US 20090012288 A1 describes the preparation of a ruthenium catalyst by the reaction of di-µ-chlorobis[(p-cymene)chlororuthenium] and N-(arylsulfonyl)-1,2-diarylethylele diamine and the use of this catalyst for the stereo-selective reduction of a protected 8-keto pentostatin derivative. However, it could not be expected offhand that a ruthenium catalyst also works in an asymmetric transfer hydrogenation performed on an unprotected derivative. Rather, it was reasonable to assume the formation of a significant amount of side products.

In a preferred embodiment, the reaction in step (ii) is catalyzed by RuCI(*p*-cymene)[(R,R)-Ts-DPEN] (i.e., [*N*-[(1*R*,2*R*)-2-(amino-κ*N*)-1,2-diphenylethyl]-4-methylbenzenesulfonamidato-κ*N*] chloro[(1,2,3,4,5,6-η)-1-methyl-4-(1-methylethyl)benzene]-ruthenium).

Particularly preferably, the reaction in step (ii) is performed in a mixture of triethylamine and formic acid, that is, under comparably mild reaction conditions. Typically, the mixture employed comprises 3 volumes of triethylamine and 1 volume of formic acid.

The reaction condition employed in step (ii) result in a shift towards a > 99% excess of the desired β-anomer.

As a result, the method of the present invention results in an up to 4-fold increase in yield of the final reaction product pentostatin as compared to methods established in the art.

The invention is further described by the figures and the following examples, which are solely for the purpose of illustrating specific embodiments of this invention, and are not to be construed as limiting the claimed subject matter in any way.

### EXAMPLES

### Example 1: Synthesis of 3-((2R,4S,5R)-4-hydroxy-5-(hydroxymethyl)-tetrahydro-furan-2-yl)-6,7-dihydroimidazo[4,5-d][1,3]diazepin-8(3H)-one

1.5 g of potassium chloride was dissolved in 0.5 l of 20 mM phosphate buffer, pH 8.0. Then, 10 g of 2'-deoxyuridine and 20 ml of nucleoside deoxyribosyltransferase solution (10 mg/ml) were added under stirring. The resulting solution was heated to 25-30°C and 2.0 g of 6,7-dihydroimidazo-[4,5-*d*][1,3]diazepin-8(3*H*)-one hydrochloride monodimethyl-sulfoxide were added. The reaction mixture was stirred at 25-30°C for 3 hours. The resulting product was purified using low-pressure reverse phase column chromatography to obtain 1.58 g (78%) of pure substance (> 99% purity as determined by HPLC).

### Example 2: Synthesis of (R)-3-((2R,4S,5R)-4-hydroxy-5-(hydroxymethyl)-tetrahydro-furan-2-yl)-3,6,7,8-tetrahydroimidazo[4,5-d][1,3]diazepin-8-ol (i.e. pentostatin).

Under N₂ atmosphere, 1.0 g of 3-((2R,4S,5R)-4-hydroxy-5-(hydroxymethyl)-tetrahydrofuran-2-yl)-6,7-dihydroimidazo[4,5-*d*][1,3]diazepin-8(3*H*)-one was added to a flask with 20 mg of RuCI(*p*-cymene)[(*R*,*R*)-Ts-DPEN]. A degassed mixture of 10 ml of triethylamine and 3.3 ml of formic acid was added. The reaction mixture was stirred one day at 40°C under N₂ purging until > 99% conversion was achieved (as determined by HPLC). Afterwards, the reaction mixture was poured into 200 ml of 100 mM phosphate buffer, pH 7.5. The resulting ratio of 8R/8S isomers of pentostatin was > 99:1 (by HPLC), after the reaction was finished. The product is purified using low-pressure reverse phase column chromatography to obtain 0.84 g (83%) of pure pentostatin with 8R/8S isomers ratio > 1000:1(by HPLC).

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. Method for the stereo-selective synthesis of the pentostatin, comprising:
(i) reacting an imidazo-[4,5-*d*]-[1,3]diazepine compound having formula (I) and a 2-deoxy-ribonucleoside, 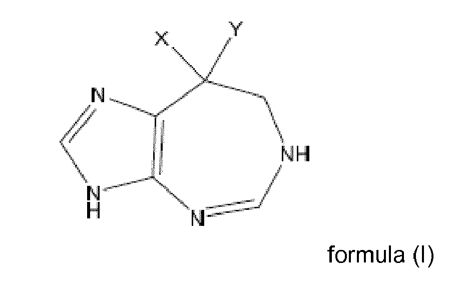 with X and Y being independently selected from H and OH or together representing a keto group =O; and
(ii) reducing the 8-keto pentostatin derivative obtained in step (i) by ruthenium-catalyzed asymmetric transfer hydrogenation;
wherein the reaction in step (i) is enzymatically catalyzed by nucleoside deoxyribosyltransferase.

2. The method of claim 1, wherein the 2-deoxy-ribonucleoside is 2-deoxy-uridine.

3. The method of claim 1 or 2, wherein the imidazo-[4,5-*d*]-[1,3]diazepine compound is 6,7-dihydroimidazo-[4,5-*d*]-[1,3]diazepin-8(3*H*)-one.

4. The method of any one of claims 1 to 3, wherein the 2-deoxy-ribonucleoside is used in an amount of 1.5 to 10 molar equivalents of the amount of the imidazo[4,5-*d*] [1,3]diazepine compound.

5. The method of claim 4, wherein the 2-deoxy-ribonucleoside is used in an amount of 4 to 7 molar equivalents of the amount of the imidazo[4,5-*d*][1,3]diazepine compound.

6. The method of any one of claims 1 to 5, wherein the reaction in step (i) is performed at a temperature between 10°C and 50°C.

7. The method of claim 6, wherein the reaction in step (i) is performed at a temperature between 25°C and 35°C.

8. The method of any one of claims 1 to 5, wherein the reaction in step (i) is performed at a pH value between 6.0 and 9.0.

9. The method of claim 8, wherein the reaction in step (i) is performed at a pH value between 7.5 and 8.0.

10. The method of any one of claims 1 to 9, wherein the reaction in step (ii) is catalyzed by RuCI(p-cymene)[(R,R)-Ts-DPEN].

11. The method of claim 10, wherein the reaction in step (ii) is performed in a mixture of triethylamine and formic acid.
